# EUROPEAN PATENT APPLICATION

(11) **EP 3 778 552 A1**
(43) Date of publication of application: **17.02.2021**
(21) Application number: 19774247.1
(22) Date of filing: 26.03.2019
(51) Int. Cl.: C07C 209/00, C07C 211/63, C07D 207/06, C07D 487/10

(54) **TETRAALKYL AMMONIUM NITRITE PRODUCTION METHOD**

(30) Priority: 26.03.2018 JP 2018057442
(71) Applicant: DAIKIN INDUSTRIES, LTD., Osaka 530-8323 (JP)
(72) Inventor: OKADA, Michiaki, Osaka-shi, Osaka 530-8323 (JP); HAYASHI, Koutarou, Osaka-shi, Osaka 530-8323 (JP); YONEDA, Satoru, Osaka-shi, Osaka 530-8323 (JP)
(74) Representative: Hoffmann Eitle
(86) International application number: PCT/JP2019/013035
(87) International publication number: WO 2019/189274

(57) **Abstract**

An object of the present disclosure is to provide a method for producing a tetraalkylammonium nitrite that is capable of efficiently producing the desired product, and/or increasing the purity of the desired product. The object is achieved by a method for producing a tetraalkylammonium nitrite including step A of reacting, in a solvent, a tetraalkylammonium salt with a metal salt that is not a silver salt, to form a tetraalkylammonium nitrite and an insoluble metal salt.

## Description

### Technical Field

The present disclosure relates to a method for producing tetraalkylammonium nitrite and related matter.

### Background Art

Tetraalkylammonium nitrite is a useful compound, for example, as a nitration agent for sulfonic acid ester.

As a method for producing a quaternary ammonium salt such as tetraalkylammonium nitrite, a method for quaternizing a tertiary amine with, for example, alkyl halide or dialkyl sulfate has been adopted.

To produce a quaternary ammonium salt with various different anionic species, a method of exchanging an anion using the obtained quaternary ammonium halide (e.g., chloride, bromide, and iodide) as a starting material is known.

Examples of these methods include the following:
(Method a) a quaternary ammonium halide is converted to a quaternary ammonium hydroxide, and an inorganic acid is added thereto to neutralize the quaternary ammonium hydroxide;
(Method b) a quaternary ammonium halide is reacted with an inorganic acid to allow the corresponding tetraalkylammonium inorganic acid salt to precipitate and separate by selecting a solvent in which the tetraalkylammonium inorganic acid salt is insoluble;
(Method c) the method disclosed in PTL 1, in which quaternary ammonium chloride is reacted with sodium sulfite, and the corresponding tetraalkylammonium sulfite is obtained by precipitation or extraction;
(Method d) the method disclosed in PTL 3, in which a quaternary ammonium halide solution is passed through an anion-exchange resin that has been ion-exchanged with nitrite ions to perform ion exchange.

However, when these methods are applied to the production of a nitrite, the following problems or drawbacks arise.

Method a and method b are difficult to use due to the instability of the corresponding nitrous acid.

Method c has a drawback in that a large amount of halogen ions remains in the obtained nitrite.

Method d is poor in productivity due to limitations such as the need for slowly passing a diluted aqueous halide solution, and distilling off water.

Thus, there is demand for a method for producing tetraalkylammonium nitrite that is capable of efficiently producing a desired product, and increasing the purity of the desired product.

### Citation List

### Patent Literature

PTL 1: US Patent No. 3175005
PTL 2: US Patent No. 3121091

### Summary of Invention

### Technical Problem

In view of the background art, an object of the present disclosure is to provide a method for producing tetraalkylammonium nitrite that is capable of efficiently producing a desired product, and/or increasing the purity of the desired product.

Another object of the present disclosure is to provide a novel tetraalkylammonium nitrite.

### Solution to Problem

The present inventors conducted extensive research, and found that the objects can be achieved by a method for producing tetraalkylammonium nitrite, comprising step A of reacting, in a polar solvent, a tetraalkylammonium salt with at least one metal nitrite selected from the group consisting of alkali metal nitrites and alkaline earth metal nitrites, to form tetraalkylammonium nitrite and an insoluble metal salt. The inventors then completed the disclosure.

The present disclosure includes the following subject matter.
Item 1. A method for producing a tetraalkylammonium nitrite, comprising step A of reacting, in a solvent, a tetraalkylammonium salt with a metal salt that is not a silver salt, to form a tetraalkylammonium nitrite and an insoluble metal salt.
Item 2. The method according to Item 1, wherein the metal salt is at least one metal nitrite selected from the group consisting of alkali metal nitrites and alkaline earth metal nitrites.
Item 3. The method according to Item 1 or 2, wherein a counter-anion of the tetraalkylammonium salt is at least one member selected from the group consisting of Cl⁻, OH⁻, BH₄⁻, HF₂⁻, Cl₂Br⁻, Br₃⁻, ClO₄-, F⁻, BF₄⁻, Br⁻, I⁻, HSO₄⁻, CH₃COO⁻, and PF₆⁻.
Item 4. The method according to Item 3, wherein the counter-anion of the tetraalkylammonium salt is at least one member selected from the group consisting of F⁻, BF₄⁻, Br⁻, I⁻, HSO₄⁻, CH₃COO⁻, and PF₆⁻.
Item 5. The method according to any one of Items 1 to 4, wherein the solvent is at least one member selected from the group consisting of non-aromatic hydrocarbon solvents, aromatic hydrocarbon solvents, halogenated hydrocarbon solvents, ether solvents, ester solvents, nitrile solvents, sulfoxide solvents, amide solvents, ketone solvents, lower alcohol solvents, and aqueous solvents.
Item 6. The method according to Item 5, wherein the solvent is at least one polar solvent selected from the group consisting of ether solvents, amide solvents, ketone solvents, and lower alcohol solvents.
Item 7. The method according to Item 6, wherein the ether solvent is represented by formula: R-O-R, wherein Rs independently represent, in each occurrence, a hydrocarbon group, or may form a hydrocarbon chain such that these hydrocarbon groups are linked to each other.
Item 8. The method according to Item 6, wherein the amide solvent is at least one member selected from the group consisting of solvents represented by formula: R-N(-COR^{A})-R, wherein Rs independently represent, in each occurrence, a hydrocarbon group, or may form a hydrocarbon chain such that these hydrocarbon groups are linked to each other, and R^{A} represents a hydrogen atom or an alkyl group.
Item 9. The method according to Item 6, wherein the ketone solvent is represented by formula: R-CO-R, wherein Rs independently represent, in each occurrence, a hydrocarbon group, or may form a hydrocarbon chain such that these hydrocarbon groups are linked to each other.
Item 10. The method according to Item 6, wherein the lower alcohol solvent is at least one member selected from the group consisting of solvents represented by formula: R-OH, wherein R represents a hydrocarbon group.
Item 11. The method according to any one of Items 1 to 10, the method producing a tetraalkylammonium nitrite represented by formula: R₄N⁺NO₂⁻, wherein Rs, in each occurrence, are identical or different, and represent a C₁-C₁₀ alkyl group optionally having at least one substituent, or two or three Rs may be taken together with an adjacent nitrogen atom to form a nitrogen-containing C₂-C₂₀ heterocyclic ring optionally having at least one substituent,
   wherein in step A, in the solvent, the tetraalkylammonium salt is reacted with the metal nitrite to form formula: Mⁿ⁺(X⁻)ₙ,
   the tetraalkylammonium salt is represented by formula: R₄N⁺X⁻, wherein X⁻ is at least one member selected from the group consisting of Cl⁻, OH⁻, BH₄⁻, HF₂⁻, Cl₂Br⁻, Br₃⁻, ClO₄⁻, F⁻, BF₄⁻, Br⁻, I⁻, HSO₄⁻, CH₃COO⁻, and PF₆⁻, preferably at least one member selected from the group consisting of F⁻, BF₄⁻, Br⁻, I⁻, HSO₄⁻, CH₃COO⁻, and PF₆⁻, and R is as defined above,
   the metal nitrite is represented by formula: Mⁿ⁺(NO₂⁻)ₙ, wherein M represents an alkali metal or an alkaline earth metal, and n represents the number 1 or 2, and
   alphabetical symbols in formula: Mⁿ⁺(X⁻)ₙ are as defined above.
Item 12. The method according to any one of Items 1 to 11, wherein the insoluble metal salt formed in step A has a solubility in water at 20°C of 1 g/100 g or less.
Item 13. The method according to any one of Items 1 to 12, further comprising step B of removing the insoluble metal salt formed in step A.
Item 14. The method according to Item 10, further comprising step C of removing the solvent.
Item 15. A tetraalkylammonium nitrite represented by formula: R₄N⁺NO₂⁻, wherein two or three Rs are taken together with an adjacent nitrogen atom to form an optionally substituted, nitrogen-containing C₂-C₂₀ heterocyclic ring, and the remaining Rs, in each occurrence, are identical or different, and represent a C₁-C₁₀ alkyl group optionally having at least one substituent.
Item 16. A composition comprising a salt,
   the salt comprising
   (a) a tetraalkylammonium nitrite, and at least one counter-cation selected from the group consisting of alkali metal nitrites and alkaline earth metal nitrites, and
   (b) at least one counter-anion selected from the group consisting of Cl⁻, OH⁻, BH₄⁻, HF₂⁻, Cl₂Br⁻, Br₃⁻, ClO₄⁻, F⁻, BF₄⁻, Br⁻, I⁻, HSO₄-, CH₃COO⁻, and PF₆⁻ .
Item 17. A composition comprising a salt,
   the salt comprising
   (a) a tetraalkylammonium nitrite, and at least one counter-cation selected from the group consisting of alkali metal nitrites and alkaline earth metal nitrites, and
   (b) at least one counter-anion selected from the group consisting of F⁻, BF⁴⁻, Br⁻, I⁻, HSO₄⁻, CH₃COO⁻, and PF₆⁻.
Item 18. The composition according to Item 16 or 17, wherein the content of the salt is less than 1 mass%.
Item 19. The composition according to any one of Items 16 to 18, wherein the content of the counter-anion is less than 1 mass%.

### Advantageous Effects of Invention

The present disclosure provides a method for producing a tetraalkylammonium nitrite that is capable of efficiently producing a desired product, and/or increasing the purity of the desired product.

The present disclosure further provides a novel tetraalkylammonium nitrite.

### Description of Embodiments

### Terms

The symbols and abbreviations in the present specification are understood, unless otherwise indicated, as indicating typical meanings in the technical field to which the present disclosure belongs, in accordance with the context of the present specification.

In the present specification, the term "insolubility" can be replaced by the phrase "insolubility or poor solubility."

In the present specification, the term "comprise" is used with the intention to include the meanings of "consist essentially of" and "consist of."

Unless otherwise indicated, the steps, treatments, and operations described in the present specification can be performed at room temperature.

In the present specification, "room temperature" refers to a temperature within the range of 10 to 40°C.

In the present specification, "Cₙ-Cₘ" (n and m represents a number) indicates that the number of carbon atoms is n or more and m or less, as would be typically understood by a person skilled in the art.

In the present specification, the term "insolubility" can indicate complete or substantial insolubility, and/or extremely low solubility. The phrase "extremely low solubility" specifically indicates, for example, that the solubility at room temperature is 1 g/100 g or less.

In the present specification, unless otherwise indicated, examples of halogen atoms include fluorine, chlorine, bromine, and iodine.

In the present specification, unless otherwise indicated, examples of alkali metals include lithium, sodium, potassium, rubidium, and cesium.

In the present specification, unless otherwise indicated, examples of alkaline earth metals include calcium, strontium, barium, and radium.

In the present specification, examples of hydrocarbyl groups include alkyl, alkenyl, alkynyl, cycloalkyl, cycloalkenyl, cycloalkadienyl, aryl, aralkyl, and groups formed of at least one combination thereof.

In the present specification, unless otherwise indicated, examples of alkyl include linear or branched C₁-C₁₀ alkyl such as methyl, ethyl, propyl (e.g., n-propyl, isopropyl), butyl (e.g., n-butyl, isobutyl, s-butyl, t-butyl), pentyl (e.g., n-pentyl, tert-pentyl, neopentyl, isopentyl, sec-pentyl, 3-pentyl), hexyl, heptyl, octyl, nonyl, and decyl.

In the present specification, unless otherwise indicated, examples of alkenyl include linear or branched C₁-C₁₀ alkenyl such as methenyl (H₂C=), vinyl, 1-propen-1-yl, 2-propen-1-yl, isopropenyl, 2-buten-1-yl, 4-penten-1-yl, and 5-hexen-1-yl.

In the present specification, unless otherwise indicated, examples of alkynyl include linear or branched C₁-C₁₀ alkynyl such as methynyl (HC≡), ethynyl, 1-propyn-1-yl, 2-propyn-1-yl, 4-pentyn-1-yl, and 5-hexyn-1-yl.

In the present specification, unless otherwise indicated, examples of alkoxy include C₁-C₆ alkoxy.

In the present specification, unless otherwise indicated, examples of C₁-C₆ alkoxy include methoxy, ethoxy, propoxy, isopropoxy, butoxy, isobutoxy, sec-butoxy, pentyloxy, and hexyloxy.

In the present specification, unless otherwise indicated, examples of cycloalkyl include C₃-C₁₀ cycloalkyl such as cyclopropyl, cyclobutyl, cyclopentyl, cyclohexyl, cycloheptyl, cyclooctyl, and adamantyl.

### 1. A Method for Producing a Tetraalkylammonium Nitrite

The method for producing a tetraalkylammonium nitrite according to the present disclosure includes step A of reacting, in a polar solvent, a tetraalkylammonium salt with at least one metal nitrite selected from the group consisting of alkali metal nitrites and alkaline earth metal nitrites to form a tetraalkylammonium nitrite and an insoluble metal salt.

The production method according to the present disclosure may further preferably include step B of removing the insoluble metal salt formed in step A.

The production method according to the present disclosure may further preferably include step C of removing the polar solvent.

### 1-1. Tetraalkylammonium Nitrite

The tetraalkylammonium nitrite produced by the production method according to the present disclosure is preferably represented by formula: R₄N⁺NO₂⁻, wherein Rs, in each occurrence, are identical or different, and represent a C₁-C₁₀ alkyl group optionally having at least one substituent, or two or three Rs may be taken together with an adjacent nitrogen atom to form a nitrogen-containing C₂-C₂₀ heterocyclic ring optionally having at least one substituent.

Rs, in each occurrence, are identical or different, and preferably represent methyl, ethyl, propyl (e.g., n-propyl, and isopropyl), butyl (e.g., n-butyl, isobutyl, s-butyl, and t-butyl), pentyl (e.g., n-pentyl, tert-pentyl, neopentyl, isopentyl, sec-pentyl, and 3-pentyl), hexyl, heptyl, or octyl.

In a preferable embodiment of the present disclosure, Rs, in each occurrence, are identical, and represent a C₁-C₄ alkyl group.

In a preferable embodiment of the present disclosure, Rs, in each occurrence, are identical, and represent methyl.

In another preferable embodiment of the present disclosure, Rs, in each occurrence, are identical, and represent ethyl.

In yet another preferable embodiment of the present disclosure, Rs, in each occurrence, are identical, and represent n-propyl.

In yet another preferable embodiment of the present disclosure, Rs, in each occurrence, are identical, and represent n-butyl.

Examples of substituents in the C₁-C₁₀ alkyl group optionally having at least one substituent, which is represented by R, include the following:
(1) a halogen atom;
(2) a nitro group;
(3) a cyano group;
(4) an amino group;
(5) an oxo group (=O);
(6) a hydroxy group;
(7) an alkoxy group optionally having at least one substituent;
(8) an optionally esterified carboxy group (e.g., alkoxy-carbonyl group optionally having at least one substituent);
(9) a thiol group;
(10) a sulfo group;
(11) a sulfamoyl group;
(12) a sulfinamoyl group;
(13) a sulfenamoyl group;
(14) a thiocarbamoyl group;
(15) an alkenyl group optionally having at least one substituent; and
(16) an alkynyl group optionally having at least one substituent.

The number of substituents may be within the range of one to the maximum number possible for substitution (e.g., 1, 2, 3, 4, 5, or 6) .

Examples of substituents that may be contained in the alkoxy group, alkenyl group, and alkynyl group include the substituents in the following substituent group A; the number of substituents may be within the range of one to the maximum number possible for substitution (e.g., 1, 2, 3, 4, 5, or 6).

Substituent group A include the following:
(a) a halogen atom;
(b) a nitro group;
(c) a cyano group;
(d) an amino group;
(e) an oxo group (=O);
(f) a hydroxy group;
(g) a thiol group;
(h) a sulfo group;
(i) a sulfamoyl group;
(j) a sulfinamoyl group;
(k) a sulfenamoyl group; and
(l) a thiocarbamoyl group.

When two or three Rs are taken together with an adjacent nitrogen atom to form a nitrogen-containing C₂-C₂₀ heterocyclic ring optionally having at least one substituent, the ring may be, for example, a 3- to 21-membered ring. The ring may further contain, as a ring-constituting atom, at least one heteroatom (e.g., nitrogen, oxygen, and sulfur) in addition to the nitrogen.

Examples of the ring include 5- to 6-membered monocyclic nitrogen-containing heterocyclic rings (e.g., pyrrolidine, and piperidine), and basket-form nitrogen-containing heterocyclic rings (e.g., quinuclidine).

Examples of substituents that may be contained in the nitrogen-containing C₂-C₂₀ heterocyclic ring include alkyl groups, and the substituents of substituent group A. The number of substituents may be within the range of one to the maximum number possible for substitution (e.g., 1, 2, 3, 4, 5, or 6).

### 1-2. Step A

Step A is explained below.

### 1-2-1. Solvent

The solvent for use in step A is preferably a polar solvent.

The solvent and the polar solvent, which is a preferable solvent, for use in step A are preferably a solvent for which the tetraalkylammonium salt shows high solubility. The solubility can be understood from the explanation regarding a tetraalkylammonium salt, described later.

The polar solvent is preferably a polar solvent for which the tetraalkylammonium nitrite shows high solubility. The solubility can be understood from the explanation regarding a tetraalkylammonium nitrite, described later.

As can be understood, for example, from the explanation regarding step B, described later, the solvent and the polar solvent, which is a preferable solvent, for use in step A are preferably a solvent for which the insoluble salt shows low solubility. The solubility can be understood from the explanation regarding an insoluble salt, described later.

As can be understood, for example, from the explanation regarding step C, described later, the polar solvent for use in step A is preferably a polar solvent that can be easily separated and removed from the tetraalkylammonium nitrite by a means such as distillation.

Other preferable properties required of the polar solvent can be understood from other descriptions in the present specification.

Examples of solvents for use in step A include non-aromatic hydrocarbon solvents, aromatic hydrocarbon solvents, halogenated hydrocarbon solvents, ether solvents, ester solvents, nitrile solvents, sulfoxide solvents, amide solvents, ketone solvents, lower alcohol solvents, and aqueous solvents.

The ether solvent may be a solvent represented by formula: R-O-R, wherein Rs, in each occurrence, independently represent a hydrocarbyl group, or may form a hydrocarbyl chain such that these hydrocarbyl groups are linked to each other.

The amide solvent may be a solvent represented by formula: R-N(-COR^{A})-R, wherein Rs, in each occurrence, independently represent a hydrocarbyl group, or may form a hydrocarbyl chain such that these hydrocarbyl groups are linked to each other, and R^{A} represents a hydrogen atom, or an alkyl group.

The ketone solvent may be a solvent represented by formula: R-CO-R, wherein Rs, in each occurrence, independently represent a hydrocarbyl group, or may form a hydrocarbyl chain such that these hydrocarbyl groups are linked to each other.

The lower alcohol solvent may be a solvent represented by formula: R-OH, wherein R represents a hydrocarbyl group, or may form a hydrocarbyl chain such that these hydrocarbyl groups are linked to each other.

The aqueous solvent may be, for example, water.

Examples of non-aromatic hydrocarbon solvents include pentane, hexane, heptane, octane, cyclohexane, decahydronaphthalene, n-decane, isododecane, and tridecane.

Examples of aromatic hydrocarbon solvents include benzene, toluene, xylene, tetralin, veratrole, diethylbenzene, methylnaphthalene, nitrobenzene, o-nitrotoluene, mesitylene, indene, and diphenyl sulfide.

Examples of ketone solvents include acetone, methyl ethyl ketone, methyl isobutyl ketone, acetophenone, propiophenone, diisobutyl ketone, and isophorone.

Examples of halogenated hydrocarbon solvents include dichloromethane, chloroform, and chlorobenzene.

Examples of ether solvents include diethyl ether, tetrahydrofuran, diisopropyl ether, methyl t-butyl ether, dioxane, dimethoxyethane, diglyme, phenetole, 1,1-dimethoxycyclohexane, and diisoamyl ether.

Examples of ester solvents include ethyl acetate, isopropyl acetate, diethyl malonate, 3-methoxy-3-methylbutyl acetate, γ-butyrolactone, ethylene carbonate, propylene carbonate, dimethyl carbonate, and α-acetyl-γ-butyrolactone.

Examples of nitrile solvents include acetonitrile and benzonitrile.

Examples of sulfoxide solvents include dimethyl sulfoxide and sulfolane.

Examples of amide solvents include N,N-dimethylformamide, N,N-dimethylacetamide, N-methylpyrrolidone, 1,3-dimethyl-2-imidazolidinone, N,N-dimethylacrylamide, N,N-dimethylacetoacetamide, N,N-diethylformamide, and N,N-diethylacetamide.

The solvent is preferably a polar solvent. Preferable examples of polar solvents for use in step A include organic polar solvents, such as
(1) lower alcohol solvents (e.g., C₁-C₆ alcohols, preferably C₁-C₄ alcohols) (specific examples: methanol, ethanol, propanol, isopropanol, and butanol),
(2) ether solvents (specific examples: tetrahydrofuran and diethyl ether),
(3) amide solvents (specific examples: N,N-dimethylformamide and N,N-dimethylacetamide), and
(4) ketone solvents (specific examples: acetone); and water.

These polar solvents may be used singly, or in a combination of two or more.

Preferable examples of polar solvents for use in step A include organic polar solvents, such as
(1) C₂-C₆ alcohol solvents (specific examples: ethanol, propanol, isopropanol, and butanol),
(2) ether solvents (specific examples: tetrahydrofuran and diethyl ether),
(3) amide solvents (specific examples: N,N-dimethylformamide and N,N-dimethylacetamide), and
(4) ketone solvents (specific examples: acetone); and water.

The polar solvent, which is a preferable solvent, may be preferably an organic polar solvent; the organic polar solvent can be a solvent that contains an organic polar solvent, a solvent that consists essentially of an organic polar solvent, or a solvent that consists of an organic polar solvent.

The organic polar solvent for use in step A may contain components other than the polar organic solvent, as long as the effects of the present disclosure are not significantly impaired. Specific examples of such other components include water.

The content of the components other than the organic polar solvent in the organic polar solvent for use in step A may be, for example, 20 mass% or less, 10 mass% or less, 5 mass% or less, 2 mass% or less, 1 mass% or less, or 0 mass%, as a total amount of the components.

The content of water as the other component may be, for example, 20 mass% or less, 10 mass% or less, 5 mass% or less, 2 mass% or less, 1 mass% or less, or 0 mass%.

The content of water in the organic polar solvent for use in step A may be, for example, 20 mass% or less, 10 mass% or less, 5 mass% or less, 2 mass% or less, 1 mass% or less, or 0 mass% as a total amount of the components.

The content of water as the other component may be, for example, 20 mass% or less, 10 mass% or less, 5 mass% or less, 2 mass% or less, 1 mass% or less, or 0 mass%.

### 1-2-2. Tetraalkylammonium Salt

The tetraalkylammonium salt to be reacted with a metal nitrite, described later, in step A is preferably represented by formula:

R₄N⁺X⁻,

wherein X⁻ is at least one member selected from the group consisting of Cl⁻, OH⁻, BH₄⁻, HF₂⁻, Cl₂Br⁻, Br₃⁻, ClO₄⁻, F⁻, BF₄⁻, Br⁻, I⁻, HSO₄⁻, CH₃COO⁻, and PF₆⁻ (preferably at least one member selected from the group consisting of F⁻, BF₄⁻, Br⁻, I⁻, HSO₄⁻, CH₃COO⁻, and PF₆⁻) . R is as defined above.

As can be understood by a person skilled in the art in view of the basic concept of the present disclosure, the tetraalkylammonium salt to be reacted with a metal nitrite, described later, is not a nitrite.

The tetraalkylammonium salt is preferably one capable of providing an insoluble metal salt that exhibits low solubility in the polar solvent, by undergoing the metal nitrite reaction in step A.

The tetraalkylammonium salt is preferably one capable of providing an insoluble metal salt that exhibits low solubility in water at 20°C, by undergoing the metal nitrite reaction in step A.

### 1-2-3. Metal Salt

The metal salt for use in the reaction with a tetraalkylammonium salt in step A as described above excludes a silver salt. The metal salt is preferably at least one metal nitrite selected from the group consisting of alkali metal nitrites and alkaline earth metal nitrites.

The metal nitrite is at least one member selected from the group consisting of alkali metal nitrites and alkaline earth metal nitrites.

Preferable examples include metal nitrites represented by formula: Mⁿ⁺(NO₂⁻)ₙ, wherein M represents an alkali metal or an alkaline earth metal, and n represents the number 1 or 2.

As would typically be understood by a person skilled in the art, n is the valence of metal M ion.

The metal nitrite formed in step A is preferably highly soluble in the polar solvent. Specifically, the solubility in the polar solvent at the temperature in step A (or 20°C) is preferably 3 g/100 g or more, more preferably 4 g/100 g or more, still more preferably 5 g/100 g or more, and yet still more preferably 6 g/100 g or more.

The metal nitrite formed in step A is preferably highly soluble in water. Specifically, the solubility in water at 20°C is preferably 953 g/100 g or more, more preferably 984 g/100 g or more, and still more preferably 995 g/100 g or more.

The upper limit of the solubility may be, although not particularly limited thereto, for example, 100 g/100g.

Specific examples of preferable metals for the metal nitrite include sodium, potassium, and calcium.

The metal nitrite is preferably one capable of providing an insoluble metal salt that exhibits low solubility in the polar solvent, by undergoing the reaction with a tetraalkylammonium salt in step A.

The metal nitrite is preferably one capable of providing an insoluble metal salt that exhibits low solubility in water at 20°C, by undergoing the reaction with a tetraalkylammonium salt in step A.

In a preferable embodiment of the production method according to the present disclosure,
in step A, in the polar solvent, a tetraalkylammonium salt wherein the anion (X⁻) is at least one member selected from the group consisting of Cl⁻, OH⁻, BH₄⁻, HF₂⁻, Cl₂Br⁻, Br₃⁻, ClO₄⁻, F⁻, BF₄⁻, Br⁻, I⁻, HSO₄⁻, CH₃COO⁻, and PF₆⁻ (preferably at least one member selected from the group consisting of F⁻, BF₄⁻, Br⁻, I⁻, HSO₄⁻, CH₃COO⁻, and PF₆⁻) is reacted with a metal nitrite represented by formula: Mⁿ⁺(NO₂⁻)ₙ, wherein M represents an alkali metal or an alkaline earth metal, and n represents the number 1 or 2, to form an insoluble metal salt represented by formula: Mⁿ⁺(X⁻)ₙ, wherein the alphabetical symbols are as defined above.

In a more preferable embodiment of the production method according to the present disclosure,
in step A, in a methanol solvent, a tetraalkylammonium salt wherein the anion (X⁻) is at least one member selected from the group consisting of Cl⁻, OH⁻, BH₄⁻, HF₂⁻, Cl₂Br⁻, Br₃⁻, ClO₄⁻, F⁻, BF₄⁻, Br⁻, I⁻, HSO₄⁻, CH₃COO⁻, and PF₆⁻ (preferably at least one member selected from the group consisting of F⁻, BF₄⁻, Br⁻, I⁻, HSO₄⁻, CH₃COO⁻, and PF₆⁻) is reacted with a metal nitrite represented by formula: Mⁿ⁺(NO₂⁻)ₙ, wherein M represents an alkali metal or an alkaline earth metal, and n represents the number 1 or 2, to form an insoluble metal salt represented by formula: Mⁿ⁺(X⁻)ₙ, wherein the alphabetical symbols are as defined above.

As can be understood from the explanation above, the combination of a polar solvent, a tetraalkylammonium salt, and a metal nitrite is preferably a combination capable of providing an insoluble metal salt that exhibits low solubility in the polar solvent.

As can be understood from the explanation above, the combination of a polar solvent, a tetraalkylammonium salt, and a metal nitrite is preferably a combination capable of providing an insoluble metal salt that exhibits low solubility in water at 20°C.

### 1-2-4. Insoluble Metal Salt

The insoluble metal salt formed in step A is a metal salt that exhibits low solubility in the polar solvent. Specifically, the insoluble metal salt is a metal salt that is insoluble in the polar solvent.

The insoluble metal salt formed in step A is a metal salt that exhibits low solubility in water. Specifically, the insoluble metal salt is a metal salt that is insoluble in water.

The solubility of the insoluble metal salt formed in step A in the polar solvent is preferably low; specifically, the solubility of the insoluble metal salt in a polar solvent at the temperature in step A (or 20°C) is preferably 0.016 g/100 g or less, more preferably 0.0088 g/100 g or less, still more preferably 0.0016 g/100 g or less, yet still more preferably 0.00088 g/100 g or less, and particularly preferably 0.00016 g/100 g.

The solubility of the insoluble metal salt formed in step A in water is preferably low; specifically, the solubility of the insoluble metal salt in water at 20°C is preferably 0.016 g/100 g or less, more preferably 0.0088 g/100 g or less, still more preferably 0.0016 g/100 g or less, yet still more preferably 0.00088 g/100 g or less, and particularly preferably 0.00016 g/100 g or less.

Due to the solubility within the ranges above, the salt is precipitated in step A; and the salt can be removed in step B, described later.

Examples of insoluble metal salts that exhibit low solubility include calcium fluoride (0.0016 g/100 g), magnesium fluoride (0.013 g/100 g), potassium tetrafluoroborate (0.45 g/100 g), sodium silicofluoride (0.44 g/100 g), and potassium silicofluoride (0.12 g/100 g). The numerical values in parentheses indicate their solubility in water at 20°C.

Specifically, examples of preferable combinations of a polar solvent, a tetraalkylammonium salt, and a metal nitrite include combinations capable of providing these insoluble metal salts.

The amount of the polar solvent for use in step A may vary depending on the solubility of the tetraalkylammonium salt. However, the amount of the polar solvent for use in step A may be, for example, preferably within the range of 1 to 1000 parts by mass, more preferably within the range of 50 to 750 parts by mass, and still more preferably within the range of 100 to 600 parts by mass, per 100 parts by mass of the tetraalkylammonium salt.

The amount of the polar solvent for use in step A may vary depending on the solubility of the metal nitrite. However, the amount of the polar solvent for use in step A may be, for example, preferably within the range of 1 to 100 parts by mass, more preferably within the range of 2 to 70 parts by mass, and still more preferably within the range of 3 to 50 parts by mass, per 100 parts by mass of the metal nitrite. The amount of the metal nitrite for use in step A may be, for example, preferably within the range of 100 to 2000 parts by mass, more preferably within the range of 200 to 1900 parts by mass, and still more preferably within the range of 300 to 1800 parts by mass, per 100 parts by mass of the tetraalkylammonium salt.

The reaction temperature in step A may be, for example, within the range of 10 to 30°C.

The reaction time in step A may be, for example, within the range of 2 to 10 hours.

Step A can be performed in the presence or absence of an inert gas (e.g., nitrogen gas).

A preferable embodiment of the production method according to the present disclosure is a method for producing a tetraalkylammonium nitrite represented by formula: R₄N⁺NO₂⁻, wherein Rs, in each occurrence, are identical or different, and represent a C₁-C₁₀ alkyl group optionally having at least one substituent, or two or three Rs may be taken together with an adjacent nitrogen atom to form a nitrogen-containing C₂-C₂₀ heterocyclic ring optionally having at least one substituent,
the method including, as step A, a step of reacting, in the polar solvent, a tetraalkylammonium salt with a metal nitrite to form an insoluble metal salt,
the tetraalkylammonium salt is represented by formula: R₄N⁺X⁻, wherein X⁻ is at least one member selected from the group consisting of Cl⁻, OH⁻, BH₄⁻, HF₂⁻, Cl₂Br⁻, Br₃⁻, ClO₄⁻, F⁻, BF₄⁻, Br⁻, I⁻, HSO₄⁻, CH₃COO⁻, and PF₆⁻ (preferably at least one member selected from the group consisting of F⁻, BF₄⁻, Br⁻, I⁻, HSO₄⁻, CH₃COO⁻, and PF₆⁻), and R is as defined above,
the metal nitrite is represented by formula: Mⁿ⁺(NO₂⁻)ₙ, wherein M represents an alkali metal or an alkaline earth metal, and n represents the number 1 or 2, and
the insoluble metal salt is represented by formula: Mⁿ⁺(X⁻)ₙ, wherein the alphabetical symbols are as defined above.

A more preferable embodiment of the production method according to the present disclosure is a method for producing a tetraalkylammonium nitrite represented by formula: R₄N⁺NO₂⁻, wherein Rs, in each occurrence, are identical or different, and represent a C₁-C₁₀ alkyl group optionally having at least one substituent, or two or three Rs are taken together with an adjacent nitrogen atom to form a nitrogen-containing C₂-C₂₀ heterocyclic ring optionally having at least one substituent,
the method including, as step A, a step of reacting, in a methanol solvent, a tetraalkylammonium salt with a metal nitrite to form an insoluble metal salt,
the tetraalkylammonium salt is represented by formula: R₄N⁺X⁻, wherein X⁻ is at least one member selected from the group consisting of Cl⁻, OH⁻, BH₄⁻, HF₂⁻, Cl₂Br⁻, Br₃⁻, ClO₄⁻, F⁻, BF₄⁻, Br⁻, I⁻, HSO₄⁻, CH₃COO⁻, and PF₆⁻ (preferably at least one member selected from the group consisting of F⁻, BF₄⁻, Br⁻, I⁻, HSO₄⁻, CH₃COO⁻, and PF₆⁻), and R is as defined above,
the metal nitrite is represented by formula: Mⁿ⁺(NO₂⁻)ₙ, wherein M represents an alkali metal or an alkaline earth metal, and n represents the number 1 or 2, and
the insoluble metal salt is represented by Mⁿ⁺(X⁻)ₙ, wherein the alphabetical symbols are as defined above.

### 1-3. Step B

The method according to the present disclosure may further include step B of removing the insoluble metal salt formed in step A.

Step B is started after the start of step A.

Step B may be performed concurrently with step A.

Another step may be performed between step A and step B.

The removal of the salt in step B may be performed by a known method, such as filtration or centrifugation.

The treatment temperature in step B can be, for example, within the range of 10 to 30°C.

Step B can be performed in the presence or absence of an inert gas (e.g., nitrogen gas).

### 1-4. Step C

The method according to the present disclosure may further include step C of removing the polar solvent.

Step C is a step of removing the polar solvent.

Step C is preferably performed after step B.

Another step may be performed between step B and step C.

The removal of the polar solvent in step C may be performed by a known method, such as distillation (e.g., distillation under reduced pressure).

The treatment temperature in step C can be, for example, within the range of 10 to 30°C.

Step C can be performed in the presence or absence of an inert gas (e.g., nitrogen gas).

The tetraalkylammonium nitrite formed in step A of the production method according to the present disclosure may be subjected to a purification step in addition to step B and step C, if desired. The purification step can be performed, for example, after step B and/or step C. The purification can be performed, for example, by using a known method capable of isolation or purification selected from commonly used methods, such as extraction, dissolution, concentration, precipitation, dehydration, adsorption, or chromatography; and a combination of these methods, as desired.

### 2. Tetraalkylammonium Compound

The tetraalkylammonium salt that can be produced by the production method according to the present disclosure and that is represented by formula: R₄N⁺NO₂⁻, is a novel compound wherein two or three Rs may be taken together with an adjacent nitrogen atom to form an optionally substituted, nitrogen-containing C₂-C₂₀ heterocyclic ring, and the remaining Rs, in each occurrence, are identical or different, and represent a C₁-C₁₀ alkyl group optionally having at least one substituent.

The present disclosure also provides this compound.

Examples of R in the formula can be the same as the examples explained for the production method.

### 3. Composition

The present disclosure also provides a composition containing a salt that contains (a) a tetraalkylammonium nitrite, and at least one counter-cation selected from the group consisting of alkali metal nitrites and alkaline earth metal nitrites, and (b) at least one counter-anion selected from the group consisting of Cl⁻, OH⁻, BH₄⁻, HF₂⁻, Cl₂Br⁻, Br₃⁻, ClO₄⁻, F⁻, BF₄⁻, Br⁻, I⁻, HSO₄⁻, CH₃COO⁻, and PF₆⁻ .

The present disclosure also provides a composition containing a salt that contains (a) a tetraalkylammonium nitrite, and at least one counter-cation selected from the group consisting of alkali metal nitrites and alkaline earth metal nitrites, and (b) at least one counter-anion selected from the group consisting of F⁻, BF⁴⁻, Br⁻, I⁻, HSO₄⁻, CH₃COO⁻, and PF₆⁻.

These compositions can be understood from the disclosures in the present specification, and common technical knowledge.

The salt content in these compositions is preferably less than 1 mass%, more preferably less than 0.5 mass%, and still more preferably less than 0.1 mass%.

The counter-anion content in these compositions is preferably less than 1%, more preferably less than 0.5 mass%, and still more preferably less than 0.1 mass%.

### Examples

The present disclosure is described below in more detail, with reference to Examples. However, the present disclosure is not limited to the Examples.

The meanings of the symbols and abbreviations in the Examples are as shown below.
THF: tetrahydrofuran
DMF: N,N-dimethylformamide
DMA: N,N-dimethylacetamide
Et₂O: diethyl ether
ACTN: acetone
EtOH: ethanol
MeOH: methanol
PrOH: propanol
iPrOH: isopropanol

### Example 1

A mixture of 1-ethyl-1-methylpyrrolidinium bromide (2.00 g), potassium nitrite (0.91 g), and methanol (24 mL) was stirred at 20°C for 2 hours. The reaction mixture was filtered under reduced pressure; and the filtrate was evaporated, thereby obtaining 1-ethyl-1-methylpyrrolidinium nitrite (1.45 g) with a yield of 90%. An internal standard substance, trifluoroethanol (0.013 g), was added to the obtained 1-ethyl-1-methylpyrrolidinium nitrite (0.1 g), and the mixture was diluted with heavy water to prepare a sample for NMR measurement. Then, ¹H-NMR measurement was performed. The obtained 1-ethyl-1-methylpyrrolidinium nitrite was confirmed to have a purity of 99% or more. Table 1 illustrates the content of impurities other than the main component. The water content of the methanol used in the reaction was measured with a Karl Fischer moisture meter (Table 1)

### Examples 2 to 19

The procedure of Example 1 was repeated to perform a reaction, thereby obtaining a tetraalkyl nitrite. 2.0 g of the compounds indicated in Table 1 (Table 1-1 and Table 1-2) were used as a starting-material ammonium salt, and 1.05 mol equivalent of potassium nitrite to the starting-material ammonium salt was used as a metal nitrite. A solvent in an amount 12 times the volume of the starting-material ammonium salt was used. The water content of each solvent used in the reaction illustrated in Table 1 (the H₂O content in a solvent (wt%)) was measured with a Karl Fischer moisture meter.

**Table 1-1**

| | | | | | | Component Other Than Main Component (Tetraalkylammonium Nitrite) | | |
|---|---|---|---|---|---|---|---|---|
| | Ammonium Salt | Solvent | H2O Content in a Solvent (wt%) | Yield (%) | Purity (%) | Starting-Material Ammonium Salt (%) | Potassium Nitrite (%) | Potassium Salt (%) |
| Example 1 | 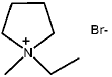 | MeOH | 0 | 95 | ≥99 | 0.2 | 0.2 | KBr 0.1 |
| Example 2 | 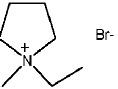 | MeOH | 1 | 95 | ≥99 | 0.2 | 0.2 | KBr 0.1 |
| Example 3 | 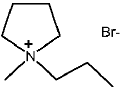 | THF | 5 | 95 | ≥99 | 0.4 | 0.1 | KBr 0.1 |
| Example 4 | 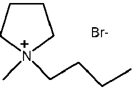 | DMF | 0 | 95 | ≥99 | 0.4 | 0.1 | KBr 0.1 |
| Example 5 | 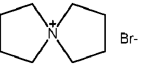 | THF | 0 | 90 | ≥99 | 0 | 0.1 | KBr 0 |
| Example 6 | 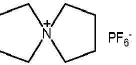 | THF | 0 | 90 | ≥99 | 0.2 | 0 | KPF6 0 |
| Example 7 | 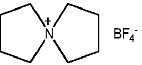 | THF | 0 | 90 | ≥99 | 0.2 | 0.4 | KBF4 0 |
| Example 8 | 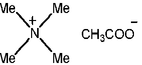 | THF | 0 | ≥99 | ≥99 | 0.1 | 0 | CH3COOK 0.2 |
| Example 9 | 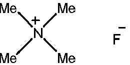 | THF | 0 | ≥99 | ≥99 | 0.3 | 0 | KF 0 |
| Example 10 | 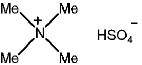 | THF | 0 | ≥99 | ≥99 | 0.2 | 0 | KHSO4 0.1 |
| Example 11 | 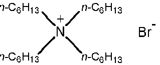 | THF | 0 | 96 | ≥99 | 0.5 | 0 | KBr 0.1 |

**Table 1-2**

| | | | | | | Component Other Than Main Component (Tetraalkylammonium Nitrite) | | |
|---|---|---|---|---|---|---|---|---|
| | Ammonium Salt | Solvent | H2O Content in a Solvent (wt%) | Yield (%) | Purity (%) | Starting-Material Ammonium Salt (%) | Potassium Nitrite (%) | Potassium Salt (%) |
| Example 12 | 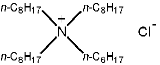 | THF | 0 | 93 | ≥99 | 0.2 | 0.1 | KC I 0.1 |
| Example 13 | 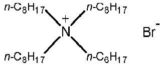 | THF | 0 | 95 | ≥99 | 0.1 | 0 | KBr 0.1 |
| Example 14 | 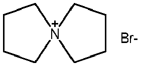 | DMA | 0 | ≥99 | ≥99 | 0.5 | 0.2 | KBr 0.1 |
| Example 15 | 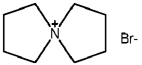 | Et₂O | 0 | ≥99 | ≥99 | 0.5 | 0 | KBr 0.2 |
| Example 16 | 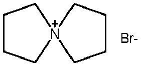 | EtOH | 0 | ≥99 | ≥99 | 0.2 | 0.1 | KBr 0.1 |
| Example 17 | 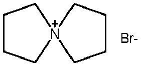 | PrOH | 0 | ≥99 | ≥99 | 0.1 | 0.1 | KBr 0 |
| Example 18 | 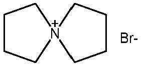 | iPrOH | 0 | ≥99 | ≥99 | 0.5 | 0 | KBr 0 |
| Example 19 | 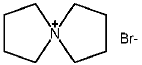 | ACTN | 0 | ≥99 | ≥99 | 0.2 | 0.1 | KBr 0.1 |

## Claims

1. A method for producing a tetraalkylammonium nitrite, comprising step A of reacting, in a solvent, a tetraalkylammonium salt with a metal salt that is not a silver salt, to form a tetraalkylammonium nitrite and an insoluble metal salt.

2. The method according to claim 1, wherein the metal salt is at least one metal nitrite selected from the group consisting of alkali metal nitrites and alkaline earth metal nitrites.

3. The method according to claim 1 or 2, wherein a counter-anion of the tetraalkylammonium salt is at least one member selected from the group consisting of Cl⁻, OH⁻, BH₄⁻, HF₂⁻, Cl₂Br⁻, Br₃⁻, ClO₄⁻, F⁻, BF₄⁻, Br⁻, I⁻, HSO₄⁻, CH₃COO⁻, and PF₆⁻.

4. The method according to claim 3, wherein the counter-anion of the tetraalkylammonium salt is at least one member selected from the group consisting of F⁻, BF₄⁻, Br⁻, I⁻, HSO₄⁻, CH₃COO⁻, and PF₆⁻.

5. The method according to any one of claims 1 to 4, wherein the solvent is at least one member selected from the group consisting of non-aromatic hydrocarbon solvents, aromatic hydrocarbon solvents, halogenated hydrocarbon solvents, ether solvents, ester solvents, nitrile solvents, sulfoxide solvents, amide solvents, ketone solvents, lower alcohol solvents, and aqueous solvents.

6. The method according to claim 5, wherein the solvent is at least one polar solvent selected from the group consisting of ether solvents, amide solvents, ketone solvents, and lower alcohol solvents.

7. The method according to claim 6, wherein the ether solvent is represented by formula: R-O-R, wherein Rs independently represent, in each occurrence, a hydrocarbon group, or may form a hydrocarbon chain such that these hydrocarbon groups are linked to each other.

8. The method according to claim 6, wherein the amide solvent is at least one member selected from the group consisting of solvents represented by formula: R-N(-COR^{A})-R, wherein Rs independently represent, in each occurrence, a hydrocarbon group, or may form a hydrocarbon chain such that these hydrocarbon groups are linked to each other, and R^{A} represents a hydrogen atom or an alkyl group.

9. The method according to claim 6, wherein the ketone solvent is represented by formula: R-CO-R, wherein Rs independently represent, in each occurrence, a hydrocarbon group, or may form a hydrocarbon chain such that these hydrocarbon groups are linked to each other.

10. The method according to claim 6, wherein the lower alcohol solvent is at least one member selected from the group consisting of solvents represented by formula: R-OH, wherein R represents a hydrocarbon group.

11. The method according to any one of claims 1 to 10, the method producing a tetraalkylammonium nitrite represented by formula: R₄N⁺NO₂⁻, wherein Rs, in each occurrence, are identical or different, and represent a C₁-C₁₀ alkyl group optionally having at least one substituent, or two or three Rs may be taken together with an adjacent nitrogen atom to form a nitrogen-containing C₂-C₂₀ heterocyclic ring optionally having at least one substituent,
wherein in step A, in the solvent, the tetraalkylammonium salt is reacted with the metal nitrite to form formula:
Mⁿ⁺(X⁻)ₙ,
the tetraalkylammonium salt is represented by formula: R₄N⁺X⁻, wherein X⁻ is at least one member selected from the group consisting of Cl⁻, OH⁻, BH₄⁻, HF₂⁻, Cl₂Br⁻, Br₃⁻, ClO₄⁻, F⁻, BF₄⁻, Br⁻, I⁻, HSO₄⁻, CH₃COO⁻, and PF₆⁻, preferably at least one member selected from the group consisting of F⁻, BF₄⁻, Br⁻, I⁻, HSO₄⁻, CH₃COO⁻, and PF₆⁻, and R is as defined above,
the metal nitrite is represented by formula: Mⁿ⁺(NO₂⁻)ₙ, wherein M represents an alkali metal or an alkaline earth metal, and n represents the number 1 or 2, and
alphabetical symbols in formula: Mⁿ⁺(X⁻)ₙ are as defined above.

12. The method according to any one of claims 1 to 11, wherein the insoluble metal salt formed in step A has a solubility in water at 20°C of 1 g/100 g or less.

13. The method according to any one of claims 1 to 12, further comprising step B of removing the insoluble metal salt formed in step A.

14. The method according to claim 10, further comprising step C of removing the solvent.

15. A tetraalkylammonium nitrite represented by formula: R₄N⁺NO₂⁻, wherein two or three Rs are taken together with an adjacent nitrogen atom to form an optionally substituted, nitrogen-containing C₂-C₂₀ heterocyclic ring, and the remaining Rs, in each occurrence, are identical or different, and represent a C₁-C₁₀ alkyl group optionally having at least one substituent.

16. A composition comprising a salt,
the salt comprising
(a) a tetraalkylammonium nitrite, and at least one counter-cation selected from the group consisting of alkali metal nitrites and alkaline earth metal nitrites, and
(b) at least one counter-anion selected from the group consisting of Cl⁻, OH⁻, BH₄⁻, HF₂⁻, Cl₂Br⁻, Br₃⁻, ClO₄⁻, F⁻, BF₄⁻, Br⁻, I⁻, HSO₄⁻, CH₃COO⁻, and PF₆⁻.

17. A composition comprising a salt,
the salt comprising
(a) a tetraalkylammonium nitrite, and at least one counter-cation selected from the group consisting of alkali metal nitrites and alkaline earth metal nitrites, and
(b) at least one counter-anion selected from the group consisting of F⁻, BF⁴⁻, Br⁻, I⁻, HSO₄⁻, CH₃COO⁻, and PF₆⁻.

18. The composition according to claim 16 or 17, wherein the content of the salt is less than 1 mass%.

19. The composition according to any one of claims 16 to 18, wherein the content of the counter-anion is less than 1 mass%.
